(19) Europäisches Patentamt
European Patent Office
Office européen des brevets

(11) Numéro de publication: **0 070 231**
**A1**

# (12) DEMANDE DE BREVET EUROPEEN

(21) Numéro de dépôt: 82401266.0

(22) Date de dépôt: 06.07.82

(51) Int. Cl.³: **C 07 D 231/12**
**A 61 K 31/415**

(30) Priorité: 15.07.81 FR 8113741

(43) Date de publication de la demande:
19.01.83 Bulletin 83/3

(84) Etats contractants désignés:
AT BE CH DE FR GB IT LI LU NL SE

(71) Demandeur: PHARMINDUSTRIE
35 Quai du Moulin de Cage
F-92231 Gennevilliers(FR)

(72) Inventeur: Bondiou, Jean-Claude
36, Place Jules Ferry
F-92120 Montrouge(FR)

(72) Inventeur: Legrand, Jean-Jacques
59 Boulevard Saint-Marcel
F-75013 Paris(FR)

(74) Mandataire: Houssin, Jean
P C U K Produits Chimiques Ugine Kuhlmann Service
Propriété Industrielle Tour Manhattan - Cedex 21
F-92087 Paris La Defense 2(FR)

## (54) Nouveau procédé de préparation de dérivés du diphényl-3,4 méthyl-5 pyrazole.

(57) Procédé de préparation des composés de formule:

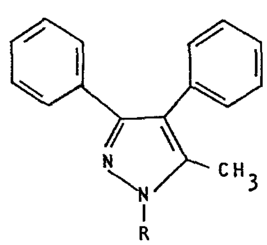

(I)

dans laquelle R est un groupe alkyle, linéaire ou ramifié, ayant 1 à 6 atomes de carbone ou un groupe phényle, caractérisé en ce que l'on fait réagir, au sein d'un solvant polaire aprotique et à une température comprise entre 0°C et la température d'ébullition du solvant, en présence d'une base, une hydrazone de formule:

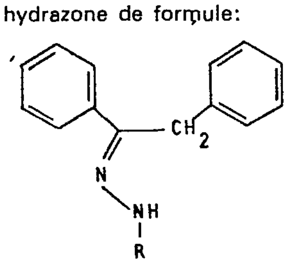

(II)

dans laquelle R a la même signification que dans la formule (I), avec un halogénure d'acétyle ou un ester de l'acide acétique de formule:

$$CH_3 - \underset{\underset{O}{\|}}{C} - X \qquad (III)$$

dans laquelle X désigne un atome d'halogène ou un groupe alcoxy bas.

Les composés de formule (I) sont utilisables en particulier pour la préparation de médicaments.

Nouveau procédé de préparation de dérivés du diphényl-3,4 méthyl-5 pyrazole

La présente invention a pour objet un nouveau procédé de préparation de dérivés du diphényl-3,4 méthyl-5 pyrazole de formule générale :

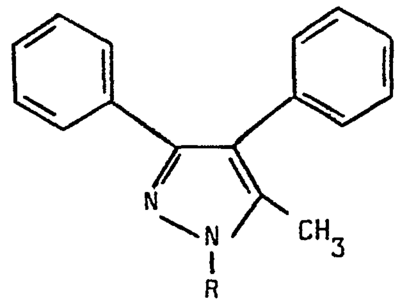

(I)

dans laquelle R représente un groupe alkyle, linéaire ou ramifié, comportant de 1 à 6 atomes de carbone ou un groupe phényle.

Il est déjà connu de préparer les composés de formule (I) par réaction, à température élevée, d'une sydnone avec un hydrocarbure acétylènique (cf. brevet américain n° 3 254 093). Il est également connu de préparer les diphényl-3,5 méthyl-1 pyrazole et diphényl-1,3 pyrazole par réaction du butyllithium avec les méthylhydrazone et phénylhydrazone dérivés de l'acétophénone, condensation des produits obtenus avec respectivement le benzoate de méthyle et le formiate de méthyle, et cyclisation en milieu acide des produits obtenus (cf. BEAM, SANDIFER, FOOTE, HAUSER, Synthetic Communications, 6 (1), 5-10, 1976).

Le procédé selon l'invention consiste à faire réagir, au sein d'un solvant polaire aprotique et en présence d'une base, à une température qui peut aller de 0°C jusqu'à la température d'ébullition du solvant et qui est de préférence de 20°C à 80°C, une hydrazone de formule (II), dans laquelle R a la même signification que dans la formule (I), avec un halogénure d'acétyle ou

un ester de l'acide acétique de formule (III), dans laquelle X désigne un atome d'halogène, de préférence un atome de chlore, ou un groupe alcoxy bas, en particulier méthoxy ou éthoxy.

La réaction peut être schématisée comme suit :

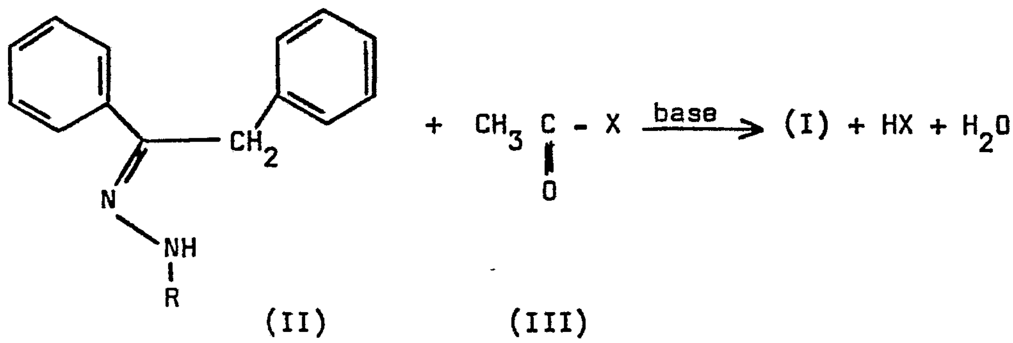


(II) (III)

Comme solvants polaires aprotiques utilisables on peut citer en particulier la N-méthylpyrrolidone et le diglyme (éther diméthylique du diéthylèneglycol). Comme bases utilisables on peut citer en particulier l'hydrure de sodium ou de potassium, l'hydroxyde de potassium et les alcoolates de métaux alcalins, particulièrement le méthylate de sodium et le tertiobutylate de potassium. Comme composé de formule (III) on emploie de préférence un halogénure d'acétyle, en particulier le chlorure d'acétyle.

Selon une première variante du procédé selon l'invention, on fait d'abord réagir l'hydrazone de formule (II) avec la base au sein du solvant polaire aprotique, à une température qui peut aller de 0°C jusqu'à la température d'ébullition du solvant et qui est de préférence de 20°C à 80°C, puis on fait réagir in situ le produit intermédiaire ainsi formé avec le composé de formule (III), à une température qui peut aller de 0°C jusqu'à la température d'ébullition du solvant et qui est de préférence de 20°C à 80°C. Dans cette première variante la base utilisée de préférence est l'hydrure de sodium ou l'hydrure

de potassium.

Selon une deuxième variante du procédé selon l'invention, on fait d'abord réagir l'hydrazone de formule (II) avec le composé de formule (III) sous forme d'halogénure d'acétyle au sein d'un solvant inerte tel qu'un hydrocarbure aromatique, en particulier le benzène ou le toluène, à une température de 0°C à 40°C et en présence d'une amine tertiaire telle que la pyridine ou la triéthylamine, puis on fait réagir le composé intermédiaire ainsi formé avec la base au sein du solvant polaire aprotique, à une température qui peut aller de 0°C jusqu'à la température d'ébullition du solvant et qui est de préférence de 20°C à 80°C. Dans cette deuxième variante la base utilisée de préférence est l'hydroxyde de sodium ou l'hydroxyde de potassium.

Les composés de formule (I) peuvent être isolés à partir des milieux réactionnels obtenus dans les procédés précédemment décrits en coulant le milieu réactionnel dans de l'eau à une température comprise entre 0°C et 20°C et en séparant le précipité formé.

Les hydrazones de formule (II) peuvent être préparées par condensation de la désoxybenzoïne avec les hydrazines de formule (IV), dans laquelle R a la même signification que dans la formule (I), selon le schéma réactionnel suivant :

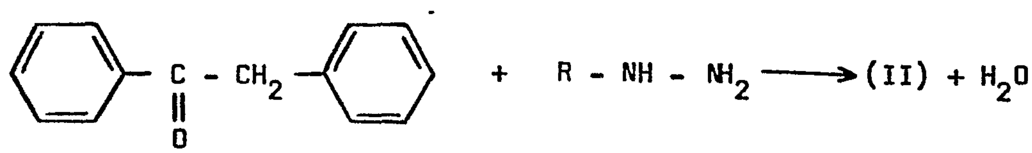

(IV)

Cette condensation peut être effectuée en particulier au sein d'un hydrocarbure aromatique tel que le benzène ou le toluène, auquel cas il n'est pas nécessaire d'isoler l'hydrazone de

formule (II) si l'on pratique le procédé selon l'invention selon sa deuxième variante.

Les dérivés du diphényl-3,4 méthyl-5 pyrazole de formule (I) sont des produits intermédiaires utilisables par exemple pour la préparation de médicaments.

Les exemples suivants illustrent l'invention sans la limiter.

<u>EXEMPLE 1</u> : Triphényl-1,3,4 méthyl-5 pyrazole

Sous atmosphère d'azote, 4,5 g d'hydrure de sodium à 80 % ($1,5 . 10^{-1}$ mole) sont mis en suspension dans 50 ml de N-méthylpyrrolidone. On chauffe à 40°C et on introduit, en 30 minutes, sous agitation, 14,25 g de la phénylhydrazone de la désoxybenzoïne ($5 . 10^{-2}$ mole) dissous dans 90 ml de N-méthylpyrrolidone. On chauffe 4 heures à 60°C sous agitation, puis on refroidit à température ambiante et on introduit, en 15 minutes, 15 ml de chlorure d'acétyle dilués dans 10 ml de N-méthylpyrrolidone. La solution rouge devient jaune. On agite 30 minutes à température ambiante, puis on coule dans 500 ml d'eau, sous agitation. On filtre le précipité obtenu et le lave par 3 fois 30 ml d'eau. On sèche sous vide jusqu'à poids constant. On obtient ainsi 14,5 g de triphényl-1,3,4 méthyl-5 pyrazole, ce qui correspond à un rendement de 93,5 %.

Après recristallisation dans l'éthanol, ce produit fond à 148°C.

<u>EXEMPLE 2</u> : Triphényl-1,3,4 méthyl-5 pyrazole

On opère comme à l'exemple 1, en partant de 2,85 g ($1 . 10^{-2}$ mole) de la phénylhydrazone de la désoxybenzoïne, 3 ml de chlorure d'acétyle, et 1,62 g ($3 . 10^{-2}$ mole) de méthylate de sodium à la place de l'hydrure de sodium. On obtient 1,25 g (ce qui correspond à un rendement de 40 %) de triphényl-1,3,4 méthyl-5

pyrazole, qui fond à 147 - 148°C.

EXEMPLE 3 : Triphényl-1,3,4 méthyl-5 pyrazole

On opère comme à l'exemple 1, en partant de 2,85 g de la phénylhydrazone de la désoxybenzoïne, 3 ml de chlorure d'acétyle, et 1,12 g de tertiobutylate de potassium à la place de l'hydrure de sodium. On obtient 1,56 g (ce qui correspond à un rendement de 50 %) de triphényl-1,3,4 méthyl-5 pyrazole, qui fond à 147-148°C.

EXEMPLE 4 : Triphényl-1,3,4 méthyl-5 pyrazole

On opère comme à l'exemple 1, en partant de 2,85 g de la phénylhydrazone de la désoxybenzoïne, 900 mg ($3 . 10^{-2}$ mole) d'hydrure de sodium à 80 %, et 3 ml d'acétate d'éthyle à la place du chlorure d'acétyle. On obtient 930 mg (ce qui correspond à un rendement de 30 %) de triphényl-1,3,4 méthyl-5 pyrazole, qui fond à 147 - 148°C.

EXEMPLE 5 : Triphényl-1,3,4 méthyl-5 pyrazole

On chauffe au reflux, pendant une heure, 25 g ($1,27 . 10^{-1}$ mole) de désoxybenzoïne et 13,7 g ($1,27 . 10^{-1}$ mole) de phénylhydrazine dans 100 ml de toluène. On refroidit à 40°C et on ajoute 14 g ($1,78 . 10^{-1}$ mole) de pyridine. On introduit alors à 40°C, en 30 minutes, 13,9 g ($1,78 . 10^{-1}$ mole) de chlorure d'acétyle. On agite 4 heures à 40°C. On ajoute 60 ml d'eau et on agite 10 minutes. On décante. La solution toluènique est concentrée sous pression réduite. L'huile obtenue est dissoute dans 175 ml de N-méthyl pyrrolidone. On ajoute 1,2 g de potasse en pastille et on agite 6 heures à température ambiante. On verse la solution rouge obtenue dans 300 ml d'eau. On essore le précipité formé. On obtient ainsi 31,2 g (ce qui correspond à un rendement de 79 % par rapport à la désoxybenzoïne) de triphényl-1,3,4 méthyl-5 pyrazole, dont le point de fusion, après recristallisation dans l'éthanol, est 148°C.

La désoxybenzoïne utilisée comme produit de départ a été préparée par le procédé de A. ROSENTHAL et M. YALPANI, Can. J. Chem. 43 (12), 3449 - 3451, (1965).

EXEMPLE 6 : Diméthyl-1,5-diphényl-3,4 pyrazole

Sous atmosphère d'azote, 4 g d'hydrure de sodium à 80 % ($1,3 . 10^{-1}$ mole) sont mis en suspension dans 30 ml de N-méthyl pyrrolidone. On ajoute à 40°C, sous agitation, en 30 minutes, 9 g ($4 . 10^{-2}$ mole) de la méthylhydrazone de la désoxybenzoïne en solution dans 60 ml de N-méthyl pyrrolidone. On chauffe 4 heures, sous agitation, à 60°C. Puis, après avoir refroidi à température ambiante, on ajoute en 15 minutes 9 ml de chlorure d'acétyle en solution dans 10 ml de N-méthyl pyrrolidone. La solution jaune obtenue est alors versée, sous agitation, dans 500 ml d'eau. On essore le précipité obtenu, le lave par 3 fois 30 ml d'eau et le sèche sous vide. On obtient ainsi 5 g de diméthyl-1,5 diphényl-3,4 pyrazole, qui fond à 122°C, ce qui correspond à un rendement de 55 %.

La méthylhydrazone de la désoxybenzoïne utilisée comme produit de départ a été préparée par le procédé décrit par Th. KAUFFMANN et Coll. Angew. Chem. 72, 752, (1960).

Revendications de brevet

1. Procédé de préparation des composés de formule :

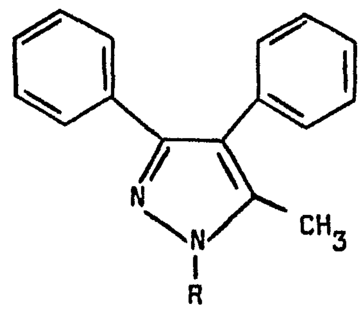

(I)

dans laquelle R est un groupe alkyle, linéaire ou ramifié, ayant 1 à 6 atomes de carbone ou un groupe phényle, caractérisé en ce que l'on fait réagir, au sein d'un solvant polaire aprotique et à une température comprise entre 0°C et la température d'ébullition du solvant, en présence d'une base, une hydrazone de formule :

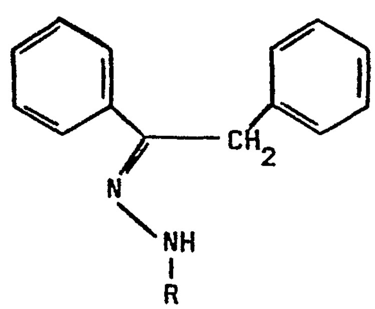

(II)

dans laquelle R a la même signification que dans la formule (I), avec un halogénure d'acétyle ou un ester de l'acide acétique de formule :

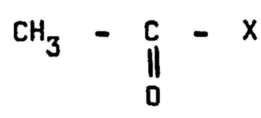

(III)

dans laquelle X désigne un atome d'halogène ou un groupe alcoxy bas.

2. Procédé selon la revendication 1, caractérisé en ce que la réaction est effectuée à une température de 20°C à 80°C.

3. Procédé selon la revendication 1, caractérisé en ce que l'on fait d'abord réagir l'hydrazone de formule (II) avec la base au sein du solvant polaire aprotique, à une température comprise entre 0°C et la température d'ébullition du solvant, puis fait réagir in situ le produit intermédiaire ainsi formé avec le composé de formule (III), à une température comprise entre 0°C et la température d'ébullition du solvant.

4. Procédé selon la revendication 3, caractérisé en ce que, dans les deux étapes de la réaction, la température est de 20°C à 80°C.

5. Procédé selon l'une quelconque des revendications 3 et 4, caractérisé en ce que la base utilisée est l'hydrure de sodium ou l'hydrure de potassium.

6. Procédé selon la revendication 1, caractérisé en ce que l'on fait d'abord réagir l'hydrazone de formule (II) avec le composé de formule (III) sous forme d'halogénure d'acétyle au sein d'un hydrocarbure aromatique, à une température de 0°C à 40°C et en présence d'une amine tertiaire, puis fait réagir le composé intermédiaire ainsi formé avec la base au sein du solvant polaire aprotique, à une température comprise entre 0°C et la température d'ébullition du solvant.

7. Procédé selon la revendication 6, caractérisé en ce que, dans la deuxième étape de la réaction, la température est de 20°C à 80°C.

8. Procédé selon l'une quelconque des revendications 6 et 7, caractérisé en ce que la base utilisée est l'hydroxyde de sodium ou l'hydroxyde de potassium.

9. Procédé selon l'une quelconque des revendications 1 à 5, caractérisé en ce que le composé de formule (III) utilisé est un halogénure d'acétyle.

10. Procédé selon l'une quelconque des revendications 6 à 9, caractérisé en ce que l'halogénure d'acétyle utilisé est le chlorure d'acétyle.

11. Procédé selon l'une quelconque des revendications 1 à 10, caractérisé en ce que le solvant polaire aprotique est la N-méthylpyrrolidone ou le diglyme.

Office européen des brevets

**0070231**

Numéro de la demande

## RAPPORT DE RECHERCHE EUROPEENNE

EP 82 40 1266

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | FR-A-1 562 151 (J.R.GEIGY S.A.) *Page 3* | 1 | C 07 D 231/12<br>A 61 K 31/415 |
| A | GB-A-1 373 212 (J.WYETH AND BROTHER) *Page 2* | 1 | |
| A | CHEMISTRY OF HETEROCYCLIC COMPOUNDS, vol. 22, 1967, pages 20-22, R.H.Wiley, Interscience, New York (USA); "Syntheses from alpha-halocarbonyl compounds with mono. and Di-thiocarbohydrazides". | | |
| | | | **DOMAINES TECHNIQUES RECHERCHES (Int. Cl. 3)** |
| | | | C 07 D 231/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 13-09-1982 | BRIGHENTI L.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : *théorie ou principe à la base de l'invention*
E : document de brevet antérieur, mais publié à la date de dépôt ou après cette date
D : cité dans la demande
L : cité pour d'autres raisons

& : membre de la même famille, document correspondant

OEB Form 1503. 03.82